Europäisches Patentamt

European Patent Office

Office européen des brevets

(11) Numéro de publication : **0 427 604 B1**

# (12) FASCICULE DE BREVET EUROPEEN

(45) Date de publication du fascicule du brevet :
**21.04.93 Bulletin 93/16**

(51) Int. Cl.$^5$ : **C07C 19/08,** C09K 5/00,
C09K 3/30, C08J 9/14,
A62D 1/00

(21) Numéro de dépôt : **90403117.6**

(22) Date de dépôt : **05.11.90**

(54) **Nouveau mélange azéotropique à bas point d'ébullition à base de fluoroalcanes et ses applications.**

(30) Priorité : **10.11.89 FR 8914788**
**08.06.90 FR 9007153**

(43) Date de publication de la demande :
**15.05.91 Bulletin 91/20**

(45) Mention de la délivrance du brevet :
**21.04.93 Bulletin 93/16**

(84) Etats contractants désignés :
**AT BE CH DE DK ES FR GB GR IT LI LU NL SE**

(56) Documents cités :
**US-A- 3 607 755**
**RESEARCH DISCLOSURE, octobre 1977, page
70, disclosure no. 16265, Industrial Opportunities Ltd, Homewell, Havant, Hampshire, GB;
"Fluorocarbon azeotropes"**

(73) Titulaire : **ELF ATOCHEM S.A.**
**4 & 8, Cours Michelet La Défense 10**
**F-92800 Puteaux (FR)**

(72) Inventeur : **Arnaud, Didier**
**12 Rue Camille Saint-Saens**
**F-92400 Courbevoie (FR)**
Inventeur : **Tanguy, Jean-Claude**
**7 Rue du Lieutenant Keiser**
**F-95110 Sannois (FR)**
Inventeur : **Sallet, Daniel**
**13 Rue Dupont de l'Eure**
**F-27470 Serquigny (FR)**

(74) Mandataire : **Leboulenger, Jean et al**
**ATOCHEM Département Propriété Industrielle**
**F-92091 Paris la Défense 10 Cédex 42 (FR)**

EP 0 427 604 B1

Jouve, 18, rue Saint-Denis, 75001 PARIS

## Description

La présente invention concerne un mélange de fluoroalcanes à bas point d'ébullition, n'ayant pas ou peu d'action sur l'environnement et utilisable pour remplacer les chlorofluorocarbures (CFC) dans les systèmes de réfrigération basse température à compression et pour remplacer le trifluorobromométhane et le difluorochlorobromométhane comme agent extincteur.

Il est maintenant établi qu'à cause de leur coefficient important d'action sur l'ozone, les CFC devront, à plus ou moins longue échéance, être remplacés par des fluides frigorigènes contenant moins de chlore et, de ce fait, moins agressifs vis-à-vis de l'environnement. Le 1,1,1,2-tétrafluoroéthane (R 134a), compte tenu de sa très faible action sur l'environnement, a déjà été proposé comme substitut aux CFC. Cependant, en raison de son point d'ébullition élevé (-26,5°C),l'utilisation du R 134a seul est réservée aux applications à température d'évaporation moyenne (-25°C ; -26°C) et ne peut pas être envisagée pour les applications à basses températures d'ébullition (-40°C par exemple). En effet, la température minimale atteinte à l'évaporateur est, dans la pratique, limitée par la valeur de la température d'ébullition normale du fluide frigorigène afin d'éviter l'introduction d'air ou de saumure dans l'installation en cas de fuites à l'évaporateur, ce qui risquerait de perturber le fonctionnement normal du système.

Dans le domaine de l'extinction et de la lutte contre l'incendie, on utilise essentiellement des chlorobromofluoroalcanes et des bromofluoroalcanes, tout particulièrement le trifluorobromométhane, le difluorochlorobromométhane et le 1,1,2,2-tétrafluoro 1,2-dibromoéthane. Ces composés sont notamment utilisés dans les locaux où se trouvent des appareillages électriques et électroniques sensibles à la corrosion (salles informatiques, centraux téléphoniques, salles de machine à bord des navires). Cependant, comme les CFC, ces composés possèdent des ODP (ozone depletion potential) élevés.

Il a maintenant été trouvé que le 1,1,1,2-tétrafluoroéthane (R 134a) et le perfluoropropane (R 218) forment un azéotrope à point d'ébullition minimum égal à environ -41,1°C à 1,013 bars et dont la teneur en R 218 au point d'ébullition normal est d'environ 76 % en masse. Bien entendu, cette composition varie en fonction de la pression du mélange et, à une pression donnée, peut être facilement déterminée suivant des techniques bien connues.

L'azéotrope selon l'invention a l'avantage de présenter un ODP nul. Cela signifie qu'il est dépourvu d'effet destructeur vis-à-vis de la couche d'ozone stratosphérique. L'ODP est défini comme le rapport entre l'abaissement de la colonne d'ozone enregistrée lors de l'émission d'une masse unitaire de substance et le même abaissement pour le trichlorofluorométhane choisi comme référence (ODP = 1). A titre indicatif le trifluorobromométhane a un ODP de 10.

Du fait de son bas point d'ébullition, le mélange azéotropique selon l'invention peut être utilisé comme fluide frigorigène dans les applications aux basses températures d'ébullition (-40°C ; -41°C) comme dans le cas de la réfrigération commerciale basse température où le R 218 seul possède de médiocres propriétés thermodynamiques et où le R 134a seul ne peut pas être utilisé pour les raisons exposées ci-dessus.

Il a également été trouvé que cet azéotrope peut être utilisé comme agent extincteur en remplacement notamment du trifluorobromométhane et du difluorochlorobromométhane. Il possède en effet une valeur de Cup Burner inférieure à 10 % et par conséquent présente un pouvoir extincteur élevé.

L'azéotrope selon l'invention peut être utilisé pour lutter contre les incendies selon les mêmes techniques que le trifluorobromométhane et le difluorochlorobromométhane. Ainsi, il peut être avantageusement utilisé pour la protection des locaux par la technique dite du noyage total. Il peut être pressurisé avec des gaz inertes tels que l'azote ce qui permet d'augmenter sa vitesse de déchargement. Il peut également être employé dans les techniques de l'extincteur portable.

Etant donné ses propriétés physiques proches de celles des CFC, le mélange selon l'invention peut également être utilisé comme propulseur d'aérosols ou comme agent d'expansion des mousses plastiques.

Les exemples suivants illustrent l'invention, sans la limiter.


## EXEMPLE 1

L'azéotrope selon l'invention a été étudié expérimentalement à différentes températures par analyse, en chromatographie phase gaz, des compositions de la phase liquide et de la phase vapeur pour différents mélanges de R 134a et R 218.

Les pressions ont été mesurées avec une précision supérieure à 0,02 bar au moyen d'un manomètre HEISE. Les températures ont été mesurées à 0,02°C près au moyen d'une sonde de platine 1000 ohms.

Le Graphe 1 en annexe représente la courbe d'équilibre liquide/vapeur des mélanges R 218/R 134a, établie à la température de 20,3°C. Sur ce graphe, l'axe des abcisses indique la fraction massique en R 218 et l'axe des ordonnées la pression absolue en bars ; les signes _ correspondent aux points expérimentaux.

2

Pour chaque température, on obtient une courbe analogue à celle du Graphe 1. Par ajouts successifs de R 218 dans le R 134a, la pression développée par le mélange augmente régulièrement, puis passe par un maximum et décroît régulièrement ce qui met en évidence l'existence de l'azéotrope à point d'ébullition minimum.

Le tableau 1 suivant donne la relation pression-température pour cet azéotrope, comparée à celle des corps purs.

### TABLEAU 1

| Température (°C) | Pression absolue (bar) | | |
|---|---|---|---|
| | Azéotrope R 218/R 134a | R 134a pur | R 218 pur |
| -40,0 | 1,10 | 0,53 | 0,87 |
| 0,3 | 4,92 | 2,94 | 4,20 |
| 20,3 | 9,08 | 5,78 | 7,66 |
| 39,9 | 15,10 | 10,26 | 12,98 |

La tension de vapeur de l'azéotrope reste sur une large gamme de température supérieure à la tension de vapeur des corps purs. Ces données indiquent que le mélange reste azéotropique dans tout cet intervalle de température.

### EXEMPLE 2

La caractérisation de l'azéotrope au point normal d'ébullition a été effectuée par mesure directe de la température d'ébullition de différents mélanges R 218/R 134a au moyen d'un ébulliomètre.

Le tableau 2 résume les résultats obtenus et permet de caractériser l'azéotrope par :
- son point d'ébullition normal qui est égal à environ -41,1°C,
- sa composition massique en R 218 qui est égale à environ 76 %.

**TABLEAU 2**

| Température (°C) | Composition massique en R 218 |
|---|---|
| - 26,5 | 0 |
| - 40,4 | 70,9 |
| - 40,8 | 74,6 |
| - 41,0 | 75,2 |
| - 41,0 | 76,4 |
| - 40,9 | 78,3 |
| - 36,7 | 100,0 |

**EXEMPLE 3**

Cet exemple illustre l'utilisation de l'azéotrope selon l'invention comme fluide frigorigène.

Les performances thermodynamiques du mélange azéotropique selon l'invention ont été comparées aux performances des deux constituants seuls, dans des conditions proches de celles rencontrées dans les systèmes de réfrigération commerciale, à savoir les suivantes :

```
- température de condensation   :    +30°C
- température d'évaporation      :    -30°C
- sous-refroidissement liquide  :    - 5°C
- surchauffe des vapeurs à
  l'aspiration du compresseur    :    +15°C
```

Le tableau 3 résume les performances thermodynamiques observées dans ces conditions pour le R 134a pur, le R 218 pur et le mélange azéotropique selon l'invention.

**TABLEAU 3**

|  | Azéotrope R 218/R 134a | R 218 pur | R 134a pur |
|---|---|---|---|
| Pression évaporation (bar) | 1,69 | 1,36 | 0,85 |
| Pression condensation (bar) | 11,58 | 10,1 | 7,70 |
| Taux de compression | 6,85 | 7,43 | 9,06 |
| Puissance frigorifique ($kJ/m^3$) | 877 | 710 | 640 |
| Coefficient de performance | 2,7 | 2,4 | 3,1 |

On peut observer que le mélange azéotropique selon l'invention offre un certain nombre d'avantages sur le R 134a ou le R 218 pur, notamment :

. un taux de compression plus faible, donc une amélioration du rendement volumétrique du compresseur et par conséquent des coûts moindres d'exploitation de l'installation

. une puissance frigorifique volumétrique disponible considérablement plus élevée, ce qui pratiquement, pour une puissance frigorifique donnée, permet l'utilisation d'un compresseur plus petit que celui défini pour utiliser le R 134a ou le R 218 pur.

Cet accroissement de puissance frigorifique volumétrique disponible, dans le cas de l'azéotrope selon l'invention, permet également d'accroître de 37 % la puissance frigorifique disponible d'une installation déjà existante définie au R 134a.

**EXEMPLE 4**

Cet exemple illustre l'utilisation de l'azéotrope selon l'invention comme agent extincteur.

L'efficacité extinctrice est généralement mesurée selon la méthode dite du Cup Burner.

Cette méthode décrite dans le projet de norme ISO/DIS 7075-1 indique le pourcentage minimum de composé extincteur (mesuré en volume) dans un mélange air plus composé extincteur nécessaire pour éteindre un combustible liquide enflammé.

Plus la valeur du Cup Burner est faible, plus le composé extincteur est efficace.

Nous avons utilisé l'éthanol comme combustible liquide.

La valeur de Cup Burner pour le mélange azéotropique R 218/R 134a selon l'invention est égale à 9,5 %.

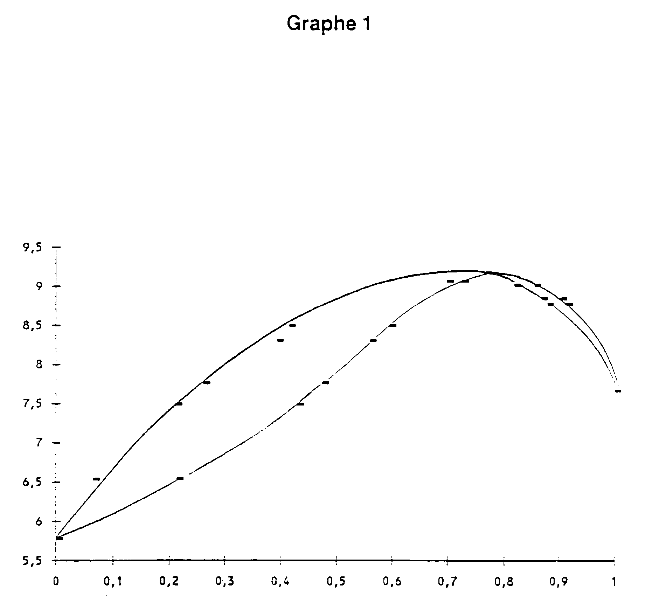

**Revendications**

1. Azéotrope à point d'ébullition minimum, caractérisé en ce qu'il consiste d'un mélange de 1,1,1,2-tétrafluoroéthane et de perfluoropropane et qu'à son point d'ébullition normale (environ -41,1°C sous 1,013 bar) il contient environ 76 % en masse de perfluoropropane et 24 % en masse de 1,1,1,2-tétrafluoroéthane.

2. Utilisation de l'azéotrope selon la revendication 1 comme fluide frigorigène.

3. Utilisation de l'azéotrope selon la revendication 1 comme propulseur d'aérosols.

4. Utilisation de l'azéotrope selon la revendication 1 comme agent d'expansion des mousses plastiques.

5. Utilisation de l'azéotrope selon la revendication 1 comme agent extincteur.

**Patentansprüche**

1. Azeotrop mit Siedepunktsminimum, dadurch gekennzeichnet, daß es aus einer Mischung von 1.1.1.2-Tetrafluorethan und von Perfluorpropan besteht und bei seinem Siedepunkt unter Normalbedingungen (ungefähr -41.1°C unter 1.013 bar) ungefähr 76 Masseprozent Perfluorpropan und 24 Masseprozent 1.1.1.2-Tetrafluorethan enthält.

2. Verwendung des Azeotrops nach Anspruch 1 als kälteerzeugende Flüssigkeit.

3. Verwendung des Azeotrops nach Anspruch 1 als Aerosoltreibmittel.

4. Verwendung des Azeotrops nach Anspruch 1 als Treibmittel für Schaumkunststoffe.

5. Verwendung des Azeotrops nach Anspruch 1 als Feuerlöschmittel.

**Claims**

1. Azeotrope with a minimum boiling point, characterised in that it consists of a mixture of 1,1,1,2-tetrafluoroethane and perfluoropropane and that at its normal boiling point (approximately -41.1°C at 1.013 bars) it contains approximately 76 % on a mass basis of perfluoropropane and 24 % on a mass basis of 1,1,1,2-tetrafluoroethane.

2. Use of the azeotrope according to Claim 1 as a refrigerating fluid.

3. Use of the azeotrope according to Claim 1 as an aerosol propellant.

4. Use of the azeotrope according to Claim 1 as a blowing agent for plastic foams.

5. Use of the azeotrope according to Claim 1 as an extinguishing agent.

# Graphe 1